# FASCICULE DE BREVET EUROPEEN

(11) **EP 0 875 538 B1**
(45) Date de publication et mention de la délivrance du brevet: **27.06.2001**
(21) Numéro de dépôt: 98400934.0
(22) Date de dépôt: 15.04.1998
(51) Int. Cl.: C09C 1/30, C09D 7/12, C08K 9/08, B32B 27/32, C08F 297/00, A61K 7/00, C08G 69/00

(54) **Silice poreuse modifiée, son procédé de fabrication et son utilisation dans les peintures et comme vecteur de pigments et colorants**
Modifizierte poröse Kieselsäure, Verfahren zu ihrer Herstellung und ihre Verwendung in Farben sowie als Träger von Pigmenten und Farbstoffen
Modified porous silica, process for preparing it and its use in paints and as carrier of pigments

(30) Priorité: 29.04.1997 FR 9705273
(43) Date de publication de la demande: 04.11.1998
(73) Titulaire: Atofina, 92800 Puteaux (FR)
(72) Inventeur: D'Herbecourt, Bruno, 27300 Bernay (FR); Julien, Olivier, 69110 Ste Foy Les Lyon (FR); Lermat, Yves, 27300 Bernay (FR); Werth, Michael, 27300 Bernay (FR)
(74) Mandataire: Neel, Henry

(56) Documents cités:
- US-A- 3 421 931
- US-A- 3 954 678
- DATABASE WPI Week 8242 Derwent Publications Ltd., London, GB; AN 82-89198E XP002046326 & JP 57 147 434 A (DAICEL CHEM. INDS.) , 11 septembre 1982
- DATABASE WPI Week 8304 Derwent Publications Ltd., London, GB; AN 83-08098K XP002046327 & JP 57 200 425 A (UNITIKA) , 8 décembre 1982

## Description

### DOMAINE TECHNIQUE

L'invention concerne le domaine des silices poreuses et particulièrement des silices poreuses modifiées utilisables notamment comme additifs de peintures liquides et comme vecteurs de pigments et/ou colorants.

### TECHNIQUE ANTERIEURE

Afin d'améliorer la résistance à l'abrasion, la résistance aux rayures des peintures, divers additifs sont ajoutés parmi lesquels on trouve notamment les poudres de silice et/ou les poudres de polyamide.

Les poudres de silice voient toutefois leur utilisation limitée en raison d'une part de la sédimentation qui se produit au sein des peintures dans lesquelles elles sont introduites, et d'autre part à cause de leur faible résistance au crayonnage (capacité d'une surface recouverte de peinture à se marquer suite au frottement d'un objet, notamment métallique -ce phénomène étant d'autant plus visible que la peinture présente une teinte claire). Pour améliorer la dispersion des poudres de silice et de limiter leur sédimentation au sein des peintures et ce, afin d'obtenir une meilleure stabilité desdites peintures, on a proposé de modifier les poudres de silice par exemple par ajout de cires de polyéthylène, mais le mélange est délicat à réaliser et cette solution technique n'apporte pas d'améliorations pour ce qui concerne les autres points faibles évoqués plus haut.

Les poudres de polyamide (PA) présentent d'excellentes propriétés de résistance à l'abrasion, à la rayure mais leur présence dans les peintures n'améliore en général pas non plus la résistance au crayonnage. En outre, le broyage de résines de polyamide à la granulométrie souhaitée -de l'ordre de quelques dizaines de µm s'avère parfois délicate lorsque lesdites poudres de PA sont obtenues par polycondensation. Des poudres de PA à la granulométrie souhaitée peuvent être obtenues directement sans broyage ultérieur par polymérisation anionique et notamment selon le procédé décrit dans la demande de brevet FR 2 619 385 ou dans le brevet EP 303 530.

Les peintures contenant poudre de silice et poudre de polyamide décrites dans la demande de brevet JP62074970 de Kansai Paint Co offrent un compromis correct mais encore insuffisant pour ce qui concerne la résistance à l'abrasion, aux rayures et au crayonnage.

### EXPOSE DE L'INVENTION

La demanderesse a maintenant trouvé des silices modifiées utilisables notamment comme additifs pour peintures (liquides ou non), comme vecteurs de colorants ou de pigments.

Les silices modifiées selon l'invention améliorent simultanément la résistance à l'abrasion, aux rayures et au crayonnage des peintures auxquelles elles sont incorporées.

L'invention concerne une silice de volume de pore compris entre 0,4 et 2 ml/g, de pouvoir d'absorption mesuré selon la norme DIN ISO 787N compris entre 100 et 350 g d'huile /100 g de silice, de diamètre de particules moyen compris entre 0,5 et 150 µm contenant des résines polyamide dans au moins une partie du volume des pores.

Le rapport massique PA/silice peut être compris entre 1/100 et 2/1.

L'art antérieur US 3421931 décrit clairement le revêtement (coating) de particules et utilise des polyamides déjà formés alors que dans la présente invention on introduit des monomères de polyamide et on forme du polyamide dans les pores par polymérisation. Dans la présente invention il y a peut-être aussi du polyamide qui enrobe la particule mais dans cet art antérieur il n'y a pas de polyamide à l'intérieur des pores. On ne voit pas comment du poylamide pourrait pénétrer dans des pores si petits, c'est pourquoi dans la présente invention on utilise des monomères de polyamide.

Le brevet US 3954678 décrit l'enrobage de gels de silice et ne décrit pas les particules utilisées dans la présente invention.

Les silices poreuses dont sont issues les silices modffiées selon l'invention ont un diamètre de particules moyen compris de préférence entre 1 et 20 µm. De telles silices poreuses peuvent être préparées par réaction d'acide sulfurique sur un silicate de sodium, puis lavage, broyage et séchage des poudres obtenues. Il est possible d'utiliser comme matière première des poudres de silice poreuse qui ont subi un enrobage organique, tel que par exemple enrobage à l'aide de cires de polyéthylène.

Les silices poreuses non enrobées sont préférées par la demanderesse.

Les silices poreuses enrobées ou non sont notamment commercialisées par la société Grace sous la marque SYLOID, et par la société J Crossfield & Sons.

Les résines polyamides qui occupent au moins une partie du volume des pores des silices modifiées selon l'invention sont des PA semi-cristallins dont le point ou le domaine de fusion est en général compris entre 80 et 275 °C ; elles sont en général préparées à partir de diacides et de diamines et/ou d'aminoacides et/ou de lactames parmi lesquels on citera tout particulièrement l'acide amino-11 undécanoïque.

La présente invention a également pour objet deux procédés de préparation des silices modifiées selon l'invention.

Le premier procédé mis au point par la demanderesse consiste à solubiliser les monomères de la résine polyamide dans une solution aqueuse ou hydroalcoolique -contenant un alcool ayant de 1 à 4 atomes de carbone. Pour la solubilisation desdits monomères, il est parfois nécessaire d'opérer à des températures supérieures à la température ambiante mais inférieures à la température d'ébullition du solvant. Une fois la solubilisation des monomères achevée, on ajoute la silice poreuse en quantité telle que toute la solution soit absorbée par la poudre de silice. On élimine le solvant en plaçant par exemple la poudre de silice dans une étuve ou dans un séchoir dans des conditions opératoires habituelles pour l'élimination d'eau ou de mélange eau/alcool (températures élevées, vide plus ou moins poussé).

On procède ensuite à la polymérisation des PA en chauffant la poudre à des températures permettant la fusion du ou des monomères de PA, typiquement comprises entre 100 et 260 °C pendant une durée variable selon la température de polymérisation choisie et en général comprise entre quelques minutes et quelques heures. Selon des techniques connues en soi, on peut accélérer la réaction de polymérisation en appliquant un vide au réacteur de polymérisation de manière à éliminer l'eau de polycondensation formée. Lors de la polymérisation des PA, on peut ajouter divers additifs (stabilisants chaleur, U.V., plastifiants, etc.), catalyseurs (de préférence choisis parmi les oxydes de phosphore), limiteurs de chaîne en général choisis parmi les mono- et diacides, les mono- et diamines, azurants, etc.

A l'issue de la polymérisation des PA on obtient la silice modifiée selon l'invention ayant sensiblement la même granulométrie que la silice poreuse de départ.

Le second procédé mis au point par la demanderesse ne peut être mis en oeuvre que pour des PA dont les monomères sont solides à température ambiante et sont susceptibles d'être réduits en poudre, par exemple par broyage. Ce second procédé consiste à mélanger à sec les monomères sous forme de poudre et la silice poreuse à la température ambiante puis à effectuer la polymérisation des monomères de PA en chauffant le mélange précédent à une température supérieure à la température de fusion desdits monomères.

A l'issue de la polymérisation, on obtient la silice modifiée selon l'invention ayant sensiblement la même granulométrie que la silice poreuse de départ.

La présente invention a également pour objet diverses utilisations de la silice modifiée décrite ci-dessus.

Elles peuvent être utilisées comme additifs de peintures pour améliorer leur résistance à l'abrasion, aux rayures et au crayonnage. En général, elles sont incorporées à raison de 0,5 à 10 % du poids total de la peinture. Comme les poudres de silice, les silices selon l'invention confèrent un effet matant à la peinture auxquelles elles sont incorporées i-e leur présence permet de diminuer notablement la brillance de la peinture.

Elles peuvent être incorporées dans des peintures liquides en phase organique ou en phase aqueuse, ou dans des peintures en poudre.

Les peintures liquides sont applicables sur les substrats à revêtir selon des techniques connues, telles que l'application à la vernisseuse en direct ou en "reverse", par projection à l'aide d'un pistolet à air mixte ou sans air, par couchage sur bande ou coil coating, etc.

Les peintures en poudre peuvent être appliquées selon des techniques variées parmi lesquelles on peut citer la projection à l'aide d'un pistolet électrostatique, l'immersion du substrat à revêtir dans un bain fluidisé.

Les peintures peuvent être appliquées sur des substrats de nature et de forme diverses. Les substrats les plus couramment utilisés sont métalliques (acier, fer, aluminium, alliages, etc.) mais on peut également revêtir des substrats en bois, verre, papier, en matériaux composites, stratifiés, laminés.

Avant enduction à l'aide de la peinture, le substrat peut subir un ou plusieurs traitements de surface (dégraissage, flammage, traitement corona, action d'un plasma, etc.) destiné à améliorer l'ancrage de la peinture ; le substrat peut être préalablement enduit à l'aide d'un primaire d'adhérence avant enduction de la peinture.

La silice modifiée selon l'invention peut également être utilisée comme agent anti-bloquant destiné à empêcher le collage sur eux-mêmes de films à base de (co)polyoléfines ou d'alliages d'oléfines qui sont pliés ou enroulés sur des bobines, comme les poudres de silice de l'art antérieur (voir par exemple DE 4 424 775).

La silice modifiée selon l'invention peut en outre être utilisée comme vecteur d'incorporation de pigments, colorants, stabilisants, antioxydants et/ou anti-U.V. et autres additifs usuels qui se présentent souvent sous forme de poudres très fines et très volatiles, ce qui a pour effet de limiter très notablement la volatilité de ces produits lors de leur manipulation et leur incorporation dans des compositions diverses sous forme liquide ou solide.

Enfin elle peut être mise en oeuvre pour la préparation de produits à usage cosmétique, dermatologique et/ou pharmaceutique, tels que poudres, ombres à paupières, rouges à lèvres, fonds de teint, produits de nettoyage ou de démaquillage, lotions, crèmes de soin, shampooings, dentifrices, etc.

### MANIERES DE REALISER L'INVENTION

### Exemples 1 à 7 préparation de silice modifiée

### Exemple 1

Dans un ballon de Rotavapor® piriforme de 500 ml, on place 30 g de silice de granulométrie moyenne 5 pm, de volume de pores 1,8 ml/g et de pouvoir d'absorption mesuré selon la norme DIN ISO 787/V égal à 320 g d'huile/100 g de silice, commercialisée par la société Grace sous la dénomination commerciale SYLOID® ED 5 ainsi que 33 g d'acide amino-11 undécanoïque (A11).

Le ballon est placé sous balayage d'azote, plongé dans un bain d'huile de silicone porté à 240 °C et mis en rotation à une vitesse de l'ordre de 1 tr/min.

Au bout de 3 min, la température au sein du ballon atteint 220 °C, c'est-à-dire supérieure à la température de fusion de l'acide amino-11 undécanoïque, et on observe un dégagement d'eau, ce qui signifie que la réaction de polycondensation a déjà commencé.

Après 30 min de réaction, la température du mélange réactionnel s'étant stabilisée à 234 °C, on refroidit le ballon tout en maintenant le balayage d'azote et on récupère une poudre de granulométrie moyenne égale à 5 µm sans agglomérat.

La viscosité inhérente du PA, extrait de la silice modifiée, mesurée dans une solution de 0,5 % de PA dans le m-crésol à 25 °C est de 0,94.

### Exemple 2

En reprenant les conditions opératoires de l'exemple 1 et à partir des mêmes produits de départ mais dans des proportions modifiées (30 parties en poids de A11 et 70 parties de silice), on prépare de la poudre de silice modifiée selon l'invention de rapport massique PA/silice 30/70.

### Exemple 3

En reprenant les conditions opératoires de l'exemple 1 et à partir des mêmes produits de départ mais dans des proportions modifiées (10 parties en poids de A11 et 90 parties de silice), on prépare de la poudre de silice modifiée selon l'invention de rapport massique PA/silice 10/90.

### Exemple 4

A partir des mêmes produits de départ que dans l'exemple 1 et dans des conditions opératoires similaires (seule la durée de polymérisation est modifiée et est portée à 1 h). On obtient une poudre de même granulométrie que la poudre de silice de départ sans agglomérat et dont le polyamide, une fois extrait de la silice modifiée, a une viscosité inhérente de 1,22.

### Exemple 5

Dans des conditions opératoires similaires à celles de l'exemple 1 (température de polymérisation : 253 °C ; durée de polymérisation : 20 min), on prépare une silice modifiée à partir de la même silice que celle de l'exemple 1, d'hexane diamine et d'acide adipique dans un rapport massique (monomères de PA-6,6)/silice de 50/50. On obtient une poudre de silice modifiée selon l'invention de même granulométrie que la poudre de silice de départ mais qui présente environ 15 % en poids d'agglomérats.

### Exemple 6

Dans des conditions opératoires similaires à celles de l'exemple 1 (température de polymérisation : 230 °C ; durée de polymérisation : 30 min), on prépare une silice modifiée à partir de la même silice que celle de l'exemple 1 et d'acide amino-6 hexanoïque dans un rapport massique acide amino-6 hexanoïque/silice de 50/50. Un tiers de l'acide amino-6 hexanoïque s'est transformé en ε-caprolactame et distille lors de la préparation de la silice modifiée. On obtient une poudre de même granulométrie que la poudre de silice de départ mais cette poudre est fortement agglomérée.

### Exemple 7

Dans des conditions opératoires similaires à celles de l'exemple 1, on essaie de préparer une silice modifiée à partir de la même silice que celle de l'exemple 1 et de dodécalactame dans un rapport massique dodécalactame/silice de 50/50. On observe la sublimation du dodécalactame et on obtient une poudre de silice dont on n'extrait pas de PA-12.

On renouvelle l'expérience en ajoutant un acide organique pour tenter de déclencher la polymérisation mais aucune amélioration n'est notée.

### Exemples 8 à 10 utilisation de silice modifiée comme additif de peinture

On prépare une peinture en préparant une dispersion des divers constituants à l'aide d'un disperseur rapide dans lequel on peut incorporer des billes de verre pour faciliter la dispersion des constituants. Une fois la dispersion effectuée, on ajoute éventuellement un ou plusieurs solvants pour obtenir une peinture à la viscosité désirée de la peinture.

| Dispersion : | (parties en poids) |
|---|---|
| - solution de résine polyuréthanne (polyester à terminaisons hydroxyles IDPI/ε-caprolactone) de viscosité mesurée à 25 °C selon la norme DIN 51757 égale à 1,06) dans un solvant constitué de xylène et d'acétate d'éthylglycol (mélange 2/1 en volume) (concentration en PU : 60 % en poids) | 74,45 |
| - mélange équivolumique d'acétate de butyldiglycol et de Solvesso®200 (mélange d'hydrocarbures commercialisé par la société Esso dont le point d'ébullition est compris entre 224 et 285 °C) | 9,60 |
| - tensioactif | 0,25 |
| - agent mouillant | 0,60 |
| - solution de dibutylaurate étain dans l'acétate de butyldiglycol (concentration en dibutylaurate étain : 40 % en poids) | 0,75 |
| - additif améliorant la résistance à l'abrasion, aux rayures et au crayonnage | 4,75 |

| Mise en viscosité : | |
|---|---|
| - mélange équivolumique d'acétate de butyldiglycol et de Solvesso®200 (mélange d'hydrocarbures commercialisé par la société Esso dont le point d'ébullition est compris entre 224 et 285 °C) | 9,60 |
| Total | 100 |

On recouvre une plaque en aluminium traité Bonder® d'épaisseur 0,8 mm à l'aide d'un primaire blanc appliqué à la barre puis réticulé pendant 40 s dans un four maintenu à 340 °C (la température maximale atteinte par le substrat étant de 240 °C). Le substrat ainsi revêtu est ensuite immergé dans de l'eau puis séché à l'air ambiant.

La peinture ci-dessus est alors appliquée à la barre sur le substrat afin d'être réticulée dans les mêmes conditions que celles mises en oeuvre pour l'application du primaire.

On obtient une matériau composite constitué :
- d'un primaire d'épaisseur égale à environ 5 µm
- d'une couche de revêtement superficiel d'épaisseur comprise entre 15 et 20 µm
- d'un substrat en aluminium d'épaisseur 0,8 mm

### Exemple 8 comparatif

Comme indiqué ci-dessus, on prépare une peinture dont l'additif améliorant la résistance à l'abrasion, aux rayures et au crayonnage est de la silice poreuse commercialisée par la société Grace sous la dénomination SYLOID® ED 5 et on recouvre un substrat à l'aide de cette peinture selon le mode opératoire indiqué plus haut.

### Exemple 9 comparatif

Comme indiqué ci-dessus, on prépare une peinture dont l'additif améliorant la résistance à l'abrasion, aux rayures et au crayonnage est un mélange de 70 parties en poids de silice poreuse SYLOID® ED 5 et 30 parties de poudre de PA-11 commercialisée par la demanderesse sous la dénomination RILSAN® D30 de granulométrie moyenne 30 µm et on recouvre un substrat à l'aide de cette peinture selon le mode opératoire indiqué plus haut.

### Exemple 10

Comme indiqué ci-dessus, on prépare une peinture dont l'additif améliorant la résistance à l'abrasion, aux rayures et au crayonnage est la silice modifiée de l'exemple 1 et on recouvre un substrat à l'aide de cette peinture selon le mode opératoire indiqué plus haut.

### Résistance à l'abrasion

On teste la résistance à l'abrasion des revêtements des exemples 8 à 10.

La résistance à l'abrasion des revêtements est mesurée à l'aide d'un abrasimètre TABER muni d'une meule CS 10 pour une charge de 500 g.

Les résultats sont réunis dans le tableau 1.

**Tableau 1**

| N° exemple | 8 | 9 | 10 |
|---|---|---|---|
| perte de poids après 800 cycles (mg) | 17,3 | 15,3 | 10,8 |
| perte de poids après 1000 cycles (mg) | ∞ | 18,8 | 13,9 |

| | | | |
|---|---|---|---|
| ∞ destruction complète du revêtement | | | |

### Stabilité au stockage

On mesure la stabilité au stockage des peintures des exemple 8 à 10 en stockant 100 g de chacune d'entre elles dans une bouteille en verre de 250 ml pendant une durée de 48 h. On évalue le dépôt éventuel formé à l'aide d'une spatule. Les résultats sont réunis dans le tableau 2.

**Tableau 2**

| N° exemple | 8 | 9 | 10 |
|---|---|---|---|
| type de dépôt | dépôt très dur, peinture difficile à réhomogénéiser | légère sédimentation, peinture facile à réhomogénéiser | légère sédimentation, peinture facile à réhomogénéiser |

### Exemples 11 à 14 utilisation de silice modifiée dans un vernis satiné (base émulsion acrylique)

### a- Préparation du vernis

On procède d'abord à la préparation d'une pâte de matage en dispersant sous agitation rapide pendant 20 min les ingrédients du Tableau 3

**Tableau 3**

| Ingrédient | Fabricant | Fonction | Parties en poids | Parties en volume |
|---|---|---|---|---|
| eau déionisée | | | 91,7 | 91,7 |
| MERGAL® K6N | Riedel-de Häen | agent fongicide | 14,8 | 14,8 |
| 1,2-propanediol | | | 341,9 | 328,8 |
| butyldiglycol | | solvant de coalescence | 171,4 | 170,4 |
| texanol | Eastman | solvant de coalescence | 136,6 | 143,8 |
| AMP ®90 | Angus Chemie GmbH | neutralisant amine | 14,8 | 15,6 |
| COATEX® RHEO 2000® | Coatex | épaississant acrylique | 28,0 | 26,4 |
| Additif∗ | | | 180,0 | 94,7 |
| BYK®-024 | Byk Chemie GmbH | agent anti-mousse | 20,8 | 23,1 |
| TOTAL | | | 1000,0 | 919,3 |

Une partie de la pâte de matage est ensuite dispersée avec les ingrédients indiqués dans le Tableau 4 pendant 20 min à une vitesse de 2.000 trs/min.

**Tableau 4**

| Ingrédient | Fabricant | Fonction | Parties en poids | Parties en volume |
|---|---|---|---|---|
| REPOLEM®2141 | Elf Atochem S.A. | émulsion acrylique | 837,5 | 797,6 |
| pâte de matage | | | cf Tab 5 | |
| eau déionisée | | | 24,5 | 24,5 |
| COATEX® RHEO 2000® | Coatex | épaississant acrylique | 12,0 | 11,3 |
| TROYSAN® POLYPHASE®AF3 | TroyChemical Corp | agent fongicide | 3,0 | 2,7 |
| BYK®-024 | BykChemie GmbH | agent anti-mousse | 2,0 | 2,1 |
| TOTAL | | | 1000,0 | 919,3 |

Dans le Tableau 5 sont indiqués les taux d'additif renforçant et la proportion de pâte de matage dans les vernis des exemples 11 à 14.

**Tableau 5**

| Exemple | Vernis | 11 | 12 | 13 | 14 |
|---|---|---|---|---|---|
| taux d'additif (% en poids) | - | 2,2 | 2,2 | 2,4 | 1,1 |
| pâte de matage(% en poids) | 0 | 12,1 | 12,1 | 24,2 | 6,05 |

Les vernis ainsi obtenus (exemples 11 à 14) ont une densité de 1,05, un pH de 8,5 et un extrait sec de 41,8 % en poids.

L'additif matant et renforçant de l'exemple 11 est du SYLOID® ED 50 (silice enrobée avec un agent organique, de granulométrie moyenne 5 µm, de volume de pores 1,8 ml/g et de pouvoir d'absorption mesuré selon la norme DIN ISO 787/V égal à 300 g d'huile/100 g de silice).

L'additif matant et renforçant des exemples 12 et 13 est la silice modifiée de l'exemple 1.

L'additif matant et renforçant de l'exemple 14 est une poudre de polyamide poreuse, de granulométrie moyenne 5 µm, de surface spécifique apparente de 10 m²/g commercialisée par la demanderesse sous la dénomination ORGASOL® 2001 UD Nat 1.

On évalue les viscosités (ICI, Brookfield) selon la norme ISO 2884, la résistance à l'abrasion TABER selon la norme NF T 30-015 et la brillance des vernis

selon la norme ISO 2813 et les résultats sont réunis dans le tableau 6.

**Tableau 6**

| Exemple | Vernis | 11 | 12 | 13 | 14 |
|---|---|---|---|---|---|
| viscosité ICI à 10⁴s⁻¹ (mPas) | 64 | 92 | 83 | 96 | 70 |
| viscosité Brookfield (mPas), axe 3, vit 20 | 380 | 1.340 | 1.620 | 3.130 | 720 |
| transparence * | 10 | 3 | 5 | 3 | 2 |
| brillant à 60° en % | 83,5 | 27 | 43,5 | 26,5 | 49,5 |
| brillant à 20° en % | 67 | 5 | 12,8 | 5 | 19 |
| abrasion TABER, (charge 500 g) | - | 30,5 | 26,3 | 24,9 | 25,7 |
| abrasion TABER (charge 1000 g) | - | 72,1 | 78,4 | 69,2 | 71,7 |
| dispersibilité | - | moyenne | bonne | bonne | mauvaise |

### Exemples 15 à 19 utilisation de silice modifiée dans une peinture en poudre comme vecteur de pigment

En reprenant le mode opératoire de l'exemple 1, on prépare une silice modifiée à partir de 50 parties en poids de SYLOID® ED 5, de 40 parties de A11 et 10 parties de pigment noir (noir de carbone commercialisé sous la dénomination MONARCH 800).

On obtient une poudre de granulométrie moyenne 5 µm dont on mélange à sec 18 parties pour 1.000 parties de poudre de PA-11 de granulométrie moyenne d'environ 100 µm commercialisée par la demanderesse sous la dénomination RILSAN® naturel grade T Nat 2 P dans un mélangeur Henschel pendant 100 s à une vitesse de 900 trs/min.

La poudre ainsi obtenue est placée dans un lit fluidisé dans des conditions d'application usuelles pour le trempage des poudres à base de PA (poudre mise en fluidisation par de l'air comprimé passant à travers la dalle poreuse du lit fluidisé). Une plaque d'acier de 10^{*}10^{*}0,3 cm préalablement chauffée pendant 10 min dans un four maintenu à 330 °C est immergée dès sa sortie du four dans le bain fluidisé pendant 6 s. On évalue visuellement l'aspect du film et mesure sa brillance selon la norme ISO 2813.

A titre de comparaison, on prépare des peintures en poudre à partir du même PA-11 auquel on ajoute de la silice non modifiée SYLOID® ED 5 et du noir de carbone MONARCH 800 (1,8 parties pour 1.000 parties de PA-11) en mélange à sec.

Par ailleurs, les formulations des peintures des exemples 15 à 19 contiennent, pour 1.000 parties de PA, 3,5 parties d'agents antioxydant et d'étalement.

On revêt des plaques d'acier de 10*10*0,3 cm à l'aide de ces peintures et évalue leur aspect et leur brillance comme indiqué plus haut. Les résultats sont réunis dans le Tableau 7.

**Tableau 7**

| N° exemple | agent matant | parties pour 1.000 de PA | Aspect du film | Brillance à 60° en % |
|---|---|---|---|---|
| 16 | silice modifiée exemple 15 | 18 | correct "rêche" au toucher | 26 |
| 17 | SYLOID® ED5 | 2 | correct | 40 |
| 18 | SYLOID® ED 5 | 5 | très nombreuses piqûres | 40 |
| 19 | SYLOID® ED 5 | 10 | non filmifié | non mesurable |

## Revendications

1. Silice de volume de pore compris entre 0,4 et 2 ml/g, de pouvoir d'absorption mesuré selon la norme DIN ISO 787N compris entre 100 et 350 g d'huile / 100 g de silice, de diamètre de particules moyen compris entre 0,5 et 150 µm contenant des résines polyamide dans au moins une partie du volume des pores.

2. Silice selon la revendication 1 dont le rapport massique PA/silice est compris entre 1/100 et 2/1,

3. Utilisation de la silice modifiée telle que définie dans l'une quelconque des revendications 1 ou 2 comme additif de vernis et/ou peintures sous forme liquide en phase organique ou en phase aqueuse ou sous forme solide.

4. Utilisation de la silice modifiée telle que définie dans l'une quelconque des revendications 1 ou 2 comme vecteur d'incorporation de composés sous forme finement divisée et/ou volatils dans des compositions liquides et/ou solides, telles que peintures, préparations cosmétiques.

5. Utilisation de la silice modifiée telle que définie dans l'une quelconque des revendications 1 ou 2 comme agents anti-bloquant pour des films à base de (co)polyoléfines ou d'alliages d'oléfines pliés ou enroulés sur des bobines.

6. Utilisation de la silice modifiée telle que définie dans l'une quelconque des revendications 1 ou 2 pour la préparation de produits à usage cosmétique, dermatologique et/ou pharmaceutique.

7. Procédé de préparation de la silice modifiée de la revendication 1, caractérisé en ce qu'on solubilise le ou les monomères des résines de polyamides dans une solution aqueuse ou hydroalcoolique, puis on ajoute la silice poreuse en quantité telle que toute la solution soit absorbée par la poudre de silice et élimine le solvant et procède à la polymérisation des monomères de PA par chauffage à une température permettant la fusion desdits monomères.

8. Procédé de préparation de la silice modifiée de la revendication 2, caractérisé en ce qu'on mélange à sec à température ambiante les monomères des résines de polyamide sous forme de poudre et la silice poreuse puis on effectue la polymérisation des monomères de PA par chauffage à une température permettant la fusion desdits monomères.

## Claims

1. Silica with a pore volume of between 0.4 and 2 ml/g, absorptivity, measured according to DIN ISO standard 787N of between 100 and 350 g of oil/100 g of silica, mean particle diameter of between 0.5 and 150 µm, containing polyamide resins in at least part of the volume of the pores.

2. Silica according to Claim 1, in which the PA/silica mass ratio is between 1/100 and 2/1.

3. Use of the modified silica as defined in either one of Claims 1 and 2 as additive to varnishes and/or paints in liquid form in organic phase or in aqueous phase or in solid form.

4. Use of the modified silica as defined in either one of Claims 1 and 2, as carrier for incorporating compounds which are in finely divided form and/or volatile in liquid and/or solid compositions such as paints and cosmetic compositions.

5. Use of the modified silica as defined in either one of Claims 1 and 2, as antiblocking agent for films based on (co)polyolefins or olefin blends, folded or wound on reels.

6. Use of the modified silica as defined in either one of Claims 1 and 2, for the preparation of products for cosmetic, dermatological and/or pharmaceutical use.

7. Process for the preparation of the modified silica of Claim 1, characterized in that the polyamide resin monomer(s) is (are) solubilized in an aqueous or aqueous-alcoholic solution and then the porous silica is added in an amount such that all the solution is absorbed by the silica powder, the solvent is removed and the polymerization of the PA monomers is carried out by heating to a temperature allowing the said monomers to melt.

8. Process for the preparation of the modified silica of Claim 2, characterized in that the polyamide resin monomers in powder form and the porous silica are mixed dry at ambient temperature and the polymerization of the PA monomers is then performed by heating to a temperature allowing the said monomers to melt.

## Patentansprüche

1. Kieselsäure mit einem Porenvolumen von 0,4 bis 2 ml/g, einem Aufnahmevermögen für Öl, das nach der Norm DIN ISO 787N gemessen wird, von 100 bis 350 g Öl pro 100 g Kieselsäure, einem mittleren Partikeldurchmesser, der im Bereich von 0,5 bis 150 µm liegt, die in mindestens einem Teil des Porenvolumens Polyamidharze enthält.

2. Kieselsäure nach Anspruch 1, wobei das Masseverhältnis der Polyamidharze zur Kieselsäure im Bereich von 1/100 bis 2/1 liegt.

3. Verwendung der wie in einem der Ansprüche 1 und 2 definierten modifizierten Kieselsäure als Additiv in Lacken und/oder Anstrichmitteln, die in flüssiger Form in einer organischen Phase oder einer wäßrigen Phase oder in fester Form vorliegen.

4. Verwendung der wie in einem der Ansprüche 1 und 2 definierten modifizierten Kieselsäure als Trägermaterial zum Einbringen von Verbindungen, die in fein zerteilter Form vorliegen und/oder flüchtig sind, in flüssige und/oder feste Zusammensetzungen, wie Anstrichmittel, kosmetische Zubereitungen.

5. Verwendung der wie in einem der Ansprüche 1 und 2 definierten modifizierten Kieselsäure als Antiblockingmittel für Filme und Folien auf der Basis von (Co)polyolefinen oder Legierungen von Olefinen, die auf Rollen gefaltet oder gewickelt sind.

6. Verwendung der wie in einem der Ansprüche 1 und 2 definierten modifizierten Kieselsäure für die Herstellung von Produkten zur kosmetischen, dermatologischen und/oder pharmazeutischen Verwendung.

7. Verfahren zur Herstellung der modifizierten Kieselsäure nach Anspruch 1, dadurch gekennzeichnet, daß das oder die Monomere der Polyamidharze in einer wäßrigen oder wäßrig-alkoholischen Lösung solubilisiert werden, anschließend die poröse Kieselsäure in einer solch großen Menge zugegeben wird, daß die gesamte Lösung von dem Kieselsäurepulver absorbiert wird, das Lösemittel entfernt wird und die Polymerisation der PA-Monomere durch Erwärmen auf eine Temperatur, bei der die Monomere zu schmelzen vermögen, durchgeführt wird.

8. Verfahren zur Herstellung der modifizierten Kieselsäure nach Anspruch 2, dadurch gekennzeichnet, daß die pulverförmigen Monomere der Polyamidharze und die poröse Kieselsäure im trockenen Zustand bei Umgebungstemperatur vermischt werden und anschließend die Polymerisation der PA-Monomere durch Erwärmen auf eine Temperatur, bei der die Monomere zu schmelzen vermögen, durchgeführt wird.
